# FASCICULE DE BREVET EUROPEEN

(11) **EP 3 371 157 B1**
(45) Date de publication et mention de la délivrance du brevet: **05.05.2021**
(21) Numéro de dépôt: 16784843.1
(22) Date de dépôt: 17.10.2016
(51) Int. Cl.: C07D 307/46

(54) **PROCEDE DE PRODUCTION DE 5-HYDROXYMETHYLFURFURAL EN PRESENCE DE CATALYSEURS DE LA FAMILLE DES ACIDES SULFONIQUES HOMOGENES EN PRESENCE D'AU MOINS UN SOLVANT POLAIRE APROTIQUE**
VERFAHREN ZUR HERSTELLUNG VON 5-HYDROXYMETHYLFURFURAL IN GEGENWART VON KATALYSATOREN DER FAMILIE HOMOGENER SULFONSÄUREN IN ANWESENHEIT VON MINDESTENS EINEM APROTISCH-POLAREN LÖSUNGSMITTEL
METHOD FOR PRODUCING 5-HYDROXYMETHYLFURFURAL IN THE PRESENCE OF CATALYSTS OF THE FAMILY OF HOMOGENEOUS SULFONIC ACIDS IN THE PRESENCE OF AT LEAST ONE APROTIC POLAR SOLVENT

(30) Priorité: 02.11.2015 FR 1560460
(43) Date de publication de la demande: 12.09.2018
(73) Titulaire: IFP Energies nouvelles, 92852 Rueil-Malmaison (FR)
(72) Inventeur: SOULEYMANOU, Myriam, 93360 Neuilly Plaisance (FR); JACQUIN, Marc, 69002 Lyon (FR); DELCROIX, Damien, 38550 St Maurice L Exil (FR)
(74) Mandataire: IFP Energies nouvelles
(86) Numéro de dépôt international: PCT/EP2016/074902
(87) Numéro de publication internationale: WO 2017/076626

(56) Documents cités:
- WO-A2-2006/063220
- US-A1- 2008 033 187
- KAWAMOTO ET AL: "Inhibition of acid-catalyzed depolymerization of cellulose with boric acid in non-aqueous acidic media", CARBOHYDRATE RESEARCH, PERGAMON, GB, vol. 343, no. 2, 7 novembre 2007 (2007-11-07), pages 249-255, XP022420337, ISSN: 0008-6215, DOI: 10.1016/J.CARRES.2007.11.004
- HÉCTOR QUIROZ-FLORENTINO ET AL: "Total synthesis of the natural succinate derivative of 5-(hydroxymethyl)furfural isolated from the Noni fruit ( Morinda citrifolia )", NATURAL PRODUCT RESEARCH, vol. 23, no. 14, 20 septembre 2009 (2009-09-20), pages 1355-1362, XP055251381, GB ISSN: 1478-6419, DOI: 10.1080/14786410903040477

## Description

### Domaine technique de l'invention

L'invention concerne un procédé de transformation de sucres et en particulier les hexoses en 5-hydroxyméthylfurfural en présence de catalyseurs de la famille des acides sulfoniques homogènes en présence d'au moins un solvant polaire aprotique.

### Art antérieur

Le 5-hydroxyméthylfurfural (5-HMF) est un composé dérivé de la biomasse qui peut être valorisé dans de nombreux domaines comme précurseur de principes actifs en pharmacie, en agrochimie ou en chimie de spécialité. Son intérêt réside ces dernières années dans son utilisation comme précurseur de l'acide furanedicarboxylique (FDCA) qui est utilisé comme substitut à l'acide téréphthalique en tant que monomère pour la production de fibres polyesters ou de plastiques de commodité.

La production de 5-HMF par déshydratation d'hexoses est connue depuis de nombreuses années et a fait l'objet d'un nombre important de travaux de recherches. D'une part, la déshydratation du glucose ou du fructose en 5-HMF est décrite en solvant polaire aprotique, par exemple le diméthylsulfoxyde DMSO ou la N-méthyl-pyrrolidone NMP, en présence de catalyseur acide hétérogène, c'est-à-dire des catalyseurs supportés insolubles dans le milieu réactionnel comme des silices sulfoniques décrites par Bao et al., Catal. Commun. 2008, 9, 1383, avec des performances correspondant à des rendements en 5-HMF d'environ 70%. D'autre part, la déshydratation du glucose ou du fructose en 5-HMF est décrite, par exemple dans les demandes de brevets US 2014/0235881, US 2014/0357878 et US 2015/0045576 en solvant polaire protique, par l'exemple l'eau ou l'éthanol, en présence de catalyseurs acides hétérogènes ou homogènes, c'est-à-dire pour ces derniers qu'ils sont solubles dans le milieu réactionnel, avec formation de sous-produits de la famille des acides carboxyliques, des esters et des éthers comme l'acide lévulinique et ses esters, l'acide formique et ses esters ainsi que les dérivés alcoxylés du 5-HMF comme le 5-éthoxyméthylfurfural. L'obtention de ces produits imposent des étapes supplémentaires de séparation et purification coûteuses nuisant à la rentabilité économique du procédé.

US2008/033187 décrit un procédé de transformation de carbohydrates, tel que le glucose ou le fructose [0011] en 5-HMF, dans un liquide ionique [0008] en présence de catalyseur de métallique ou acide [0015] tel que AICI3, CrCl2, CrCl3, FeCl2, FeCl3, CuCI, CuBr, CuCl2, CuBr2, VCl3, MoCl3, PdCl2, PtCl2, PtCl4, RuCl3, RhCl3.

WO2006/063220 décrit un un procédé amélioré de préparation de 2,5-(hydroxyméthyl) furaldéhyde comprenant: i) la combinaison d'une source de fructose, un solvant choisi dans le groupe consistant en 1-méthyl-2-pyrrolidinone, diméthylacétamide, diméthylformamide et leurs combinaisons, avec un catalyseur pour fournir un mélange réactionnel; ii) chauffer ledit mélange réactionnel à une température et pendant une durée suffisantes pour favoriser une réaction de déshydratation catalysée par un acide du fructose dans ladite source de fructose pour former un mélange de produits; et iii) isoler le 2,5- (hydroxyméthyl) furaldéhyde dudit mélange de produits.

L'article intitulé "Inhibition of acid-catalyzed depolymerization of cellulose with boric acid in non-aqueous acidic media" (CARBOHYDRATE RESEARCH, PERGAMON, GB, vol. 343, no. 2, 7 novembre 2007 (2007-11-07), pages 249-255), étudie l'influence de l'acide borique sur les réactions de déhydratation notamment via l'utilisation d'acide sulfurique en combinaison avec un acide borique dans le sulfolane.

L'article intitulé "Total synthesis of the natural succinate derivative of 5-(hydroxymethyl)furfural isolated from the Noni fruit (Morinda citrifolia)" (NATURAL PRODUCT RESEARCH, vol. 23, no. 14, 20 septembre 2009 (2009-09-20), pages 1355-1362, XP055251381) décrit une synthèse total d'une molécule naturelle à partir du 5-HMF et en en particulier l'obtention de 5-HMF à partir de glucose ou de fructose dans le DMSO en présence d'acide sulfurique.

Il existe donc un besoin de développement de nouveaux procédés de transformation sélective de sucres en 5-HMF permettant d'obtenir de meilleurs rendements en limitant la formation de sous-produits non désirés.

De manière surprenante, la demanderesse a mis en évidence que la mise en contact de sucres avec un ou plusieurs catalyseurs de la famille des acides sulfoniques homogènes en présence d'au moins un solvant polaire aprotique permettait d'augmenter significativement les rendements en 5-HMF en limitant la formation de sous-produits non désirés.

L'invention concerne donc un procédé de production de 5-hydroxyméthylfurfural à partir de sucres utilisant des catalyseurs de la famille des acides sulfoniques homogènes en présence d'au moins un solvant polaire aprotique.

### Objet de l'invention

Un objet de la présente invention est donc de fournir un nouveau procédé de transformation d'une charge comprenant au moins un sucre en 5-hydroxyméthylfurfural, dans lequel ladite charge est mise en contact avec un ou plusieurs catalyseurs de la famille des acides sulfoniques homogènes en présence d'au moins un solvant polaire aprotique dont le moment dipolaire exprimé en Debye (D) est supérieur à 2,00, choisi parmi la pyridine, la butan-2-one, l'acétone, l'anhydride acétique, la N,N,N',N'-tétraméthylurée, le benzonitrile, l'acétonitrile, la méthyléthylcétone, le propionitrile, l'hexaméthylphosphoramide, le nitrobenzène, le nitrométhane, le N,N-diméthylformamide, le N,N-diméthylacétamide, le sulfolane, la N-méthylpyrrolidone, le diméthylsulfoxyde et le propylène carbonate, pris seul ou en mélange, à une température comprise entre 30°C et 200°C, et à une pression comprise entre 0,1 MPa et 10 MPa dans lequel lesdits catalyseurs sont choisis parmi les composés de formule générale 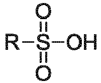 dans laquelle R est choisi parmi
- les groupements halogènes,
- les groupements alkyles linéaires ou ramifiés, cycliques ou non cycliques comprenant de 1 à 20 atomes de carbone pouvant être substitués ou non par au moins un substituant choisi parmi :
   ∘ le groupe oxo,
   ∘ les groupements halogènes et,
   ∘ les groupements aryles, fusionnés ou non, pouvant être substitués ou non par des groupements halogènes et/ou des groupements alkyles linéaires ou ramifiés, cycliques ou non cycliques comportant de 1 à 20 atomes de carbone,
- les groupements aryles comprenant de 6 à 14 atomes de carbone pouvant être substitués ou non par au moins un substituant choisi parmi :
   ∘ les groupements alkyles, linéaires ou ramifiés, cycliques ou non cycliques comprenant de 1 à 20 atomes de carbone pouvant être substitués ou non par au moins un groupement halogéné ou par au moins un groupement nitro,
   ∘ les groupements halogénés et,
   ∘ le groupement nitro.

On entend par catalyseur de la famille des acides sulfoniques, une molécule de la famille des acides sulfoniques portant une fonction acide SO₃H.

On entend par acide sulfonique homogène, un acide sulfonique soluble dans le milieu réactionnel.

On entend par solvant aprotique, une molécule jouant le rôle de solvant et dont tous les hydrogènes sont portés par des atomes de carbone.

On entend par solvant polaire, une molécule jouant le rôle de solvant dont le moment dipolaire µ exprimé en Debye a une valeur numérique supérieure ou égale à 2,00 mesuré à 25°C.

On entend donc par solvant polaire aprotique, une molécule jouant le rôle de solvant dont tous les hydrogènes sont portés par des atomes de carbone et dont le moment dipolaire µ exprimé en Debye a une valeur numérique supérieure ou égale à 2,00 mesuré à 25°C.

Un avantage de la présente invention est de fournir un procédé de transformation de sucres en 5-hydroxyméthylfurfural utilisant un ou plusieurs catalyseurs de la famille des acides sulfoniques homogènes en solvant polaire aprotique et limitant la formation de sous-produits non désirés comme les produits de la famille des acides carboxyliques, les esters, les éthers et les humines. Les humines sont des produits secondaires de condensation résultants de la dégradation des sucres en milieu acide tels que les polyfuranes.

### Description détaillée de l'invention

### La charge

La charge traitée dans le procédé selon l'invention est une charge comprenant au moins un sucre, de préférence choisi parmi les oligosaccharides et les monosaccharides, seuls ou en mélange.

Par sucre, on entend tout oligosaccharide ou monosaccharide soluble dans les conditions réactionnelles envisagées par l'invention.

Par monosaccharide, on désigne plus particulièrement les hydrates de carbone de formule générale C₆(H₂O)₆ ou C₆H₁₂O₆. Les monosaccharides préférés utilisés comme charge dans la présente invention sont choisis parmi le glucose, le mannose et le fructose, pris seuls ou en mélange.

Par oligosaccharide, on désigne plus particulièrement un hydrate de carbone ayant pour formule brute C₆ₙH₁₀ₙ₊₂O₅ₙ₊₁ où n est un entier supérieur à 1, les unités monosaccharidiques composant ledit oligosaccharide étant identiques ou non, et/ou un hydrate de carbone ayant pour formule brute (C₆ₘH₁₀ₘ₊₂O₅ₘ₊₁)(C₅ₙH₈ₙ₊₂O₄ₙ₊₁) où m et n sont des entiers supérieurs ou égaux à 1, les unités monosaccharidiques composant ledit oligosaccharide étant identiques ou non.

Les oligosaccharides sont de préférence choisis parmi les oligomères d'hexoses ou de pentoses et d'hexoses, de préférence parmi les oligomères d'hexoses, de préférence avec un degré de polymérisation leur permettant d'être soluble dans les conditions réactionnelles envisagées par l'invention. Ils peuvent être obtenus par hydrolyse partielle de polysaccharides issus de ressources renouvelables tels que l'amidon, l'inuline, la cellulose ou l'hémicellulose, éventuellement issus de la biomasse lignocellulosique. Par exemple, l'explosion à la vapeur de la biomasse lignocellulosique est un procédé d'hydrolyse partielle de la cellulose et de l'hémicellulose contenues dans la biomasse lignocellulosique produisant un flux d'oligo- et monosaccharides.

Les oligosaccharides préférés utilisés comme charge dans la présente invention sont de préférence choisis parmi le saccharose, le lactose, le maltose, l'isomaltose, l'inulobiose, le mélibiose, le gentiobiose, le tréhalose, le cellobiose, le cellotriose, le cellotetraose et les oligosaccharides issus de l'hydrolyse desdits polysaccharides issus de l'hydrolyse de l'amidon, de l'inuline, de la cellulose ou de l'hémicellulose, pris seuls ou en mélange.

De préférence, la charge comprenant au moins un sucre utilisée dans le procédé selon l'invention est choisie parmi le cellobiose, le fructose et le glucose, pris seuls ou en mélange.

De manière très préférée, ladite charge est choisie parmi le fructose et le glucose, pris seuls ou en mélange.

### Les catalyseurs

Conformément à l'invention, ladite charge est mise en contact dans le procédé selon l'invention, avec au moins un catalyseur de la famille des acides sulfoniques homogènes en présence d'au moins un solvant polaire aprotique pris seul ou en mélange, à une température comprise entre 30°C et 200°C, et à une pression comprise entre 0,1 MPa et 10 MPa.

Conformément à l'invention, le catalyseur est choisi parmi les composés acides sulfoniques homogènes de formule générale : 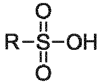 dans laquelle R est choisi parmi
- les groupements halogènes,
- les groupements alkyles, linéaires ou ramifiés, cycliques ou non cycliques comprenant de 1 à 20 atomes de carbone pouvant être substitués ou non par au moins un substituant choisi parmi :
   ∘ le groupe oxo,
   ∘ les groupements halogènes et,
   ∘ les groupements aryles, fusionnés ou non, pouvant être substitués ou non par des groupements halogènes et/ou des groupements alkyles linéaires ou ramifiés, cycliques ou non cycliques comportant de 1 à 20 atomes de carbone,
- les groupements aryles comprenant de 6 à 14 atomes de carbone pouvant être substitués ou non par au moins un substituant choisi parmi :
   ∘ les groupements alkyles, linéaires ou ramifiés, cycliques ou non cycliques comprenant de 1 à 20 atomes de carbone, pouvant être substitués ou non par au moins un groupement halogéné ou par au moins un groupement nitro,
   ∘ les groupements halogénés et,
   ∘ le groupement nitro.

Dans le cas où R est un groupement halogène, le dit groupement halogène est de préférence choisi parmi le fluor, le chlore, le brome et l'iode.

De manière préférée, dans le cas où R est un groupement halogène, ledit groupement halogène est le fluor.

Dans le cas où R est un groupement alkyle linéaire, ledit groupement alkyle linéaire comporte de 1 à 20 atomes de carbone de préférence 1 à 10 atomes de carbone et de manière préférée 1 à 6 atomes de carbone.

De manière encore plus préférée, dans le cas où R est un groupement alkyle linéaire, ledit groupement alkyle linéaire est choisi parmi les groupements méthyle, éthyle et propyle.

De manière très avantageuse, dans le cas où R est un groupement alkyle linéaire, ledit groupement alkyle linéaire est le méthyle et le catalyseur de la famille des acides sulfoniques est l'acide méthanesulfonique.

Dans le cas où R est un groupement alkyle ramifié, ledit groupement alkyle ramifié comporte de 3 à 20 atomes de carbone, de préférence 3 à 10 atomes de carbone et de manière préférée 3 à 6 atomes de carbone.

De manière encore plus préférée, dans le cas où R est un groupement alkyle ramifié, ledit groupement alkyle ramifié est choisi parmi les groupements isopropyle, isobutyle et tertbutyle.

Dans le cas où R est un groupement alkyle cyclique, ledit groupement alkyle cyclique comporte de 3 à 20 atomes de carbone, de préférence 5 à 8 atomes de carbone.

De manière préférée, dans le cas où R est un groupement alkyle cyclique, ledit groupement alkyle cyclique est choisi parmi les groupements cyclopentyle et cyclohexyle.

Dans le cas où R est un groupement alkyle substitué par au moins un groupe oxo (=O) ledit groupe oxo peut être positionné sur un carbone terminal ou non. Ledit groupe oxo pouvant ainsi faire partie d'une fonction cétone, aldéhyde ou acide carboxylique.

De manière préférée, dans le cas ou R est un groupement alkyle substitué par au moins un groupe oxo (=O), ledit groupe oxo fait partie d'une fonction cétone ou d'une fonction aldéhyde.

Dans le cas où R est un groupement alkyle substitué par au moins un groupement halogène, ledit groupement halogène est de préférence choisi parmi le fluor, le chlore, le brome et l'iode et de préférence le fluor.

De manière très préférée, dans le cas où R est un groupement alkyle substitué par au moins un groupement halogène, R est le trifluorométhyle et le catalyseur de la famille des acides sulfoniques est l'acide trifluorométhanesulfonique.

Dans le cas où R est un groupement alkyle substitué par au moins un groupement aryle, ledit groupement aryle est avantageusement choisi parmi le phényle, le tolyle et le naphtyle.

De manière préférée, dans le cas où R est un groupement alkyle substitué par au moins un groupement aryle, ledit groupement aryle est le phényle et R est le groupement benzyle.

Dans le cas où R est un groupement alkyle substitué par au moins un groupement aryle, ledit groupement alkyle est avantageusement substitué par au moins un groupement halogène choisi parmi le fluor, le chlore, le brome et l'iode, de préférence le fluor.

Dans le cas où R est un groupement aryle, ledit groupement aryle comporte de 6 à 14 atomes de carbone, de préférence 6 à 10 atomes de carbone.

De manière préférée, dans le cas où R est un groupement aryle, le dit groupement aryle est le phényle ou le naphtyle.

Dans le cas où R est un groupement aryle substitué par au moins un groupement halogène, ledit groupement halogène est de préférence choisi parmi le fluor, le chlore, le brome et l'iode et de manière préférée le fluor.

Dans le cas où R est un groupement aryle substitué par au moins un groupement alkyle, ledit groupement alkyle est avantageusement choisi parmi les alkyles linéaires ou ramifiés comportant de 1 à 6 atomes de carbone.

De manière préférée, dans le cas où R est un groupement aryle substitué par au moins un groupement alkyle, ledit groupement alkyle est choisi parmi le méthyle, l'éthyle, le propyle et l'isopropyle.

De manière encore plus préférée, dans le cas où R est un groupement aryle substitué par au moins un groupement alkyle, ledit groupement alkyle est le méthyle et le catalyseur de la famille des acides sulfoniques est l'acide paratoluène sulfonique.

Dans le cas où R est un groupement aryle substitué par un groupement alkyle, ledit groupement alkyle est avantageusement substitué par au moins un groupement halogène choisi parmi le fluor, le chlore, le brome et l'iode, de préférence le fluor.

### Procédé de transformation

Conformément à l'invention, le procédé de transformation de la charge comprenant au moins un sucre est mis en oeuvre dans une enceinte réactionnelle en présence d'au moins un solvant, ledit solvant étant un solvant polaire aprotique ou un mélange de solvants polaires aprotiques, à une température comprise entre 30°C et 200°C, et à une pression comprise entre 0,1 MPa et 10 MPa.

Le procédé est donc mis en œuvre dans une enceinte réactionnelle comprenant au moins un solvant polaire aprotique et dans laquelle ladite charge est mise en présence d'au moins un catalyseur de la famille des acides sulfoniques selon l'invention.

Conformément à l'invention, le procédé opère en présence d'au moins un solvant, ledit solvant étant un solvant polaire aprotique ou un mélange de solvants polaires aprotiques.

Les solvants polaires aprotiques sont choisis parmi tous les solvants polaires aprotiques dont le moment dipolaire exprimé en Debye (D) est supérieur ou égal à 2,00. Les solvants polaires aprotiques sont choisis parmi la pyridine (2,37), la butan-2-one (5,22), l'acétone (2,86), l'anhydride acétique (2,82), la *N,N,N',N'*-tétraméthylurée (3,48), le benzonitrile (4,05), l'acétonitrile (3,45), la méthyléthylcétone (2,76), le propionitrile (3,57), l'hexaméthylphosphoramide (5,55), le nitrobenzène (4,02), le nitrométhane (3,57), le *N,N-*diméthylformamide (3,87), le *N,N*-diméthylacétamide (3,72), le sulfolane (4,80), la *N*-méthylpyrrolidone (4,09), le diméthylsulfoxyde (3,90), le propylène carbonate (4,94) et la γ-valérolactone (4,71) seuls ou en mélange.

De préférence, les solvants polaires aprotiques sont avantageusement choisis parmi l'acétone, le *N,N-*diméthylformamide, le *N,N*-diméthylacétamide, le sulfolane, la *N*-méthylpyrrolidone, le diméthylsulfoxyde, le propylène carbonate et la γ-valérolactone seuls ou en mélange.

De manière préférée, les solvants polaires aprotiques sont avantageusement choisis parmi le *N,N-*diméthylacétamide, la *N*-méthylpyrrolidone, le diméthylsulfoxyde, et la γ-valérolactone seuls ou en mélange.

De préférence, ledit procédé selon l'invention opère à une température comprise entre 50°C et 200°C et de manière préférée entre 50°C et 175°C, et à une pression comprise entre 0,1 MPa et 8 MPa et de manière préférée entre 0,1 et 5 MPa.

Généralement le procédé peut être opéré selon différents modes de réalisation. Ainsi, le procédé peut avantageusement être mis en œuvre en discontinu ou en continu. On peut opérer dans une enceinte réactionnelle fermée ou en réacteur semi-ouvert.

Le ou les catalyseurs de la famille des acides sulfoniques homogènes sont introduits dans l'enceinte réactionnelle à raison d'une quantité correspondant à un rapport massique charge/catalyseur(s) compris entre 1 et 1000, de préférence entre 1 et 500, de préférence entre 1 et 200, de préférence entre 1 et 150.

La charge est introduite dans le procédé à raison d'une quantité correspondant à un rapport massique solvant/charge compris entre 0,1 et 200, de préférence entre 0,3 et 100 et encore préférentiellement entre 1 et 50.

Si on choisit un procédé en continu, la vitesse massique horaire (débit de charge massique/masse de catalyseur(s)) est comprise entre 0,01 h⁻¹ et 5 h⁻¹, de préférence entre 0,02 h⁻¹ et 2 h⁻¹.

A la fin de la réaction, le catalyseur peut être facilement récupéré par précipitation, distillation, extraction ou lavage. Il peut être également récupéré par passage sur une résine échangeuse d'ion comme l'Amberlyst 31 et recyclé après lavage de cette résine.

### Les produits obtenus et leur mode d'analyse

Le produit de la réaction du procédé de transformation selon l'invention est le 5-hydroxyméthylfurfural (5-HMF).

A l'issue de la réaction, le milieu réactionnel est analysé par chromatographie phase gaz (GC) pour déterminer la teneur en 5-HMF en présence d'un étalon interne et par chromatographie ionique pour déterminer la conversion de la charge en présence d'un étalon externe et pour quantifier les produits non désirés comme l'acide lévulinique, l'acide formique, les alcoxyméthylfurfural et les humines.

### EXEMPLES

Dans les exemples ci-dessous, le glucose et le fructose utilisés comme charge sont commerciaux et utilisés sans purification supplémentaire.

L'Amberlyst 15 et l'acide méthanesulfonique noté AMS dans les exemples sont commerciaux et utilisés sans purification supplémentaire.

Le diméthylsulfoxyde, noté DMSO dans les exemples et la *N*-méthylpyrrolidone, notée NMP dans les exemples, utilisés comme solvant polaire aprotique, sont commerciaux et utilisés sans purification supplémentaire.

L'éthanol utilisé comme solvant polaire protique est commercial et utilisé sans purification supplémentaire.

Pour les exemples 1 à 8 de transformation de sucres en 5-HMF, le rendement molaire en 5-HMF est calculé par le rapport entre le nombre de moles de 5-HMF obtenu et le nombre de moles de charge sucre engagé.

### Exemple 1 : Transformation du fructose mettant en œuvre l'acide méthanesulfonique dans la NMP (conforme)

L'acide méthanesulfonique (0,018 g, 0,19 mmol) est ajouté à une solution de fructose (2,0 g, 11,10 mmol) dans la NMP (20 g). Le rapport massique charge/catalyseur est de 111. Le rapport massique solvant/charge est de 10. Le milieu réactionnel est ensuite agité à 120 °C pendant 6 h. La conversion du fructose en 5-HMF est suivi par des prélèvements réguliers d'un aliquot de solution qui est refroidi instantanément à 0°C, redissous dans l'eau et contrôlé par chromatographie ionique. Le rendement molaire en 5-HMF après 6h est de 78%. Le rendement en humines non désirées est de 22%.

### Exemple 2 : Transformation d'un mélange de glucose et fructose mettant en œuvre l'acide méthanesulfonique dans la NMP (conforme)

L'acide méthanesulfonique (0,018 g, 0,19 mmol) est ajouté à un mélange de fructose et de glucose 50% poids/50% poids (2,0 g, 11,10 mmol) dans la NMP (20 g). Le rapport massique charge/catalyseur est de 111. Le rapport massique solvant/charge est de 10. Le milieu réactionnel est ensuite agité à 120 °C pendant 6 h. La conversion du mélange de fructose et de glucose en 5-HMF est suivi par des prélèvements réguliers d'un aliquot de solution qui est refroidi instantanément à 0°C, redissous dans l'eau et contrôlé par chromatographie ionique. Le rendement molaire en 5-HMF après 6h est de 70%. Le rendement en humines non désirées est de 30%.

### Exemple 3 : Transformation du fructose mettant en œuvre l'acide méthanesulfonique dans le DMSO (conforme)

L'acide méthanesulfonique (0,018 g, 0,19 mmol) est ajouté à une solution de fructose (2,0 g, 11,10 mmol) dans le DMSO (20 g). Le rapport massique charge/catalyseur est de 111. Le rapport massique solvant/charge est de 10. Le milieu réactionnel est ensuite agité à 120 °C pendant 6 h. La conversion du fructose en 5-HMF est suivi par des prélèvements réguliers d'un aliquot de solution qui est refroidi instantanément à 0°C, redissous dans l'eau et contrôlé par chromatographie ionique. Le rendement molaire en 5-HMF après 6h est de 82%. Le rendement en humines non désirées est de 18%.

### Exemple 4 : Transformation d'un mélange de glucose et fructose mettant en œuvre l'acide méthanesulfonique dans le DMSO (conforme)

L'acide méthanesulfonique (0,018 g, 0,19 mmol) est ajouté à un mélange de fructose et de glucose 50% poids/50% poids (2,0 g, 11,10 mmol) dans le DMSO (20 g). Le rapport massique charge/catalyseur est de 111. Le rapport massique solvant/charge est de 10. Le milieu réactionnel est ensuite agité à 120 °C pendant 6 h. La conversion du mélange de fructose et de glucose en 5-HMF est suivi par des prélèvements réguliers d'un aliquot de solution qui est refroidi instantanément à 0°C, redissous dans l'eau et contrôlé par chromatographie ionique. Le rendement molaire en 5-HMF après 6h est de 76%. Le rendement en humines non désirées est de 24%.

### Exemple 5 comparatif : Transformation de fructose sans catalyseur dans la NMP (non conforme)

Le fructose (2,0 g, 11,10 mmol) est dissous dans la NMP (20 g). Le rapport massique solvant/charge est de 10. Le milieu réactionnel est ensuite agité à 120 °C pendant 6 h. La conversion du fructose en 5-HMF est suivi par des prélèvements réguliers d'un aliquot de solution qui est refroidi instantanément à 0°C, redissous dans l'eau et contrôlé par chromatographie ionique. Le rendement molaire en 5-HMF après 6h est inférieur à 1%. Le rendement en humines non désirées est de 40%.

### Exemple 6 comparatif : Transformation de fructose sans catalyseur dans le DMSO (non conforme)

Le fructose (2,0 g, 11,10 mmol) est dissous dans le DMSO (20 g). Le rapport massique solvant/charge est de 10. Le milieu réactionnel est ensuite agité à 120 °C pendant 6 h. La conversion du fructose en 5-HMF est suivi par des prélèvements réguliers d'un aliquot de solution qui est refroidi instantanément à 0°C, redissous dans l'eau et contrôlé par chromatographie ionique. Le rendement molaire en 5-HMF après 6h est de 65%. Le rendement en humines non désirées est de 35%.

### Exemple 7 comparatif : Transformation de fructose mettant en œuvre un catalyseur acide sulfonique hétérogène Amberlyst 15 dans la NMP (non conforme)

L'Amberlyst 15 (0,040 g, 0,19 mmol) est ajouté à une solution de fructose (2,0 g, 11,10 mmol) dans la NMP (20 g). Le rapport massique charge/catalyseur est de 50. Le rapport massique solvant/charge est de 10. Le milieu réactionnel est ensuite agité à 120 °C pendant 6 h. La conversion du fructose en 5-HMF est suivi par des prélèvements réguliers d'un aliquot de solution qui est refroidi instantanément à 0°C, redissous dans l'eau et contrôlé par chromatographie ionique. Le rendement molaire en 5-HMF après 6h est de 45%. Le rendement en humines non désirées est de 55%.

### Exemple 8 comparatif : Transformation de fructose mettant en œuvre l'acide méthanesulfonique dans l'éthanol, solvant polaire protique (non conforme)

L'acide méthanesulfonique (0,018 g, 0,19 mmol) est ajouté à une solution de fructose (2,0 g, 11,10 mmol) dans l'éthanol (20 g). Le rapport massique charge/catalyseur est de 111. Le rapport massique solvant/charge est de 10. Le milieu réactionnel est ensuite agité à 120 °C pendant 6 h. La conversion du fructose en 5-HMF est suivi par des prélèvements réguliers d'un aliquot de solution qui est refroidi instantanément à 0°C, redissous dans l'eau et contrôlé par chromatographie ionique. Le rendement molaire en 5-HMF après 6h est de 17%. Les rendements en lévulinate d'éthyle, 5-éthoxyméthylfurfural et humines non désirés sont respectivement de 7%, 5% et 37%.

Les résultats présentant le rendement en 5-HMF lors du prélèvement réalisé après 6 heures de réaction sont récapitulés dans le Tableau 1.

**Tableau 1**

| Exemple | Charge | Catalyseur | Solvant | Rendement 5-HMF (%) | Rendement produits non désirés (%) |
|---|---|---|---|---|---|
| 1 (conforme) | Fructose | AMS | NMP | 78 | Humines 22 |
| 2 (conforme) | Glucose +Fructose | AMS | NMP | 70 | Humines 30 |
| 3 (conforme) | Fructose | AMS | DMSO | 82 | Humines 18 |
| 4 (conforme) | Glucose +Fructose | AMS | DMSO | 76 | Humines 24 |
| 5 (non conforme) | Fructose | - | NMP | <1 | Humines 40 |
| 6 (non conforme) | Fructose | - | DMSO | 65 | Humines 35 |
| 7 (non conforme) | Fructose | Amberlyst 15 | NMP | 45 | Humines 55 |
| 8 (non conforme) | Fructose | AMS | Ethanol | 17 | Lévulinate d'éthyle 7 Ethoxyméthylfurfural 5 Humines 37 |

La cinétique de réaction est plus rapide et le rendement en 5-HMF est supérieur dans le cas de l'utilisation de catalyseurs de la famille des acides sulfoniques homogènes comme l'acide méthanesulfonique dans un solvant polaire aprotique selon l'invention comparativement à l'absence de catalyseurs dans un solvant polaire aprotique et comparativement à l'utilisation d'un catalyseur de la famille des acides sulfoniques hétérogènes comme l'Amberlyst 15 dans un solvant polaire aprotique et comparativement à l'utilisation de catalyseurs de la famille des acides sulfoniques homogènes comme l'acide méthanesulfonique dans un solvant polaire protique comme l'éthanol.

Le rendement en produits non désirés est inférieur dans le cas de l'utilisation de catalyseurs de la famille des acides sulfoniques homogènes comme l'acide méthanesulfonique dans un solvant polaire aprotique selon l'invention comparativement à l'absence de catalyseurs dans un solvant polaire aprotique et comparativement à l'utilisation d'un catalyseur de la famille des acides sulfoniques hétérogènes comme l'Amberlyst 15 dans un solvant polaire aprotique et comparativement à l'utilisation de catalyseurs de la famille des acides sulfoniques homogènes comme l'acide méthanesulfonique dans un solvant polaire protique comme l'éthanol.

Il paraît donc de manière inattendue qu'il est nettement avantageux d'utiliser des catalyseurs de la famille des acides sulfoniques homogènes dans un solvant polaire aprotique selon l'invention pour la transformation de sucres en 5-HMF.

## Revendications

1. Procédé de transformation d'une charge comprenant au moins un sucre en 5-hydroxyméthylfurfural, dans lequel ladite charge est mise en contact avec un ou plusieurs catalyseurs de la famille des acides sulfoniques homogènes en présence d'au moins un solvant polaire aprotique dont le moment dipolaire exprimé en Debye (D) est supérieur à 2,00, choisi parmi la pyridine, la butan-2-one, l'acétone, l'anhydride acétique, la N,N,N',N'-tétraméthylurée, le benzonitrile, l'acétonitrile, la méthyléthylcétone, le propionitrile, l'hexaméthylphosphoramide, le nitrobenzène, le nitrométhane, le N,N-diméthylformamide, le N,N-diméthylacétamide, le sulfolane, la N-méthylpyrrolidone, le diméthylsulfoxyde et le propylène carbonate, pris seul ou en mélange, à une température comprise entre 30°C et 200°C, et à une pression comprise entre 0,1 MPa et 10 MPa dans lequel lesdits catalyseurs sont choisis parmi les composés de formule générale 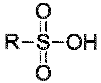 dans laquelle R est choisi parmi
• les groupements halogènes,
• les groupements alkyles linéaires ou ramifiés, cycliques ou non cycliques comprenant de 1 à 20 atomes de carbone pouvant être substitués ou non par au moins un substituant choisi parmi :
∘ le groupe oxo,
∘ les groupements halogènes et,
∘ les groupements aryles, fusionnés ou non, pouvant être substitués ou non par des groupements halogènes et/ou des groupements alkyles linéaires ou ramifiés, cycliques ou non cycliques comportant de 1 à 20 atomes de carbone,
• les groupements aryles comprenant de 6 à 14 atomes de carbone pouvant être substitués ou non par au moins un substituant choisi parmi :
∘ les groupements alkyles, linéaires ou ramifiés, cycliques ou non cycliques comprenant de 1 à 20 atomes de carbone pouvant être substitués ou non par au moins un groupement halogéné ou par au moins un groupement nitro,
∘ les groupements halogénés et,
∘ le groupement nitro.

2. Procédé selon la revendication 1 dans lequel ledit sucre est choisi parmi les oligosaccharides et les monosaccharides, seuls ou en mélange.

3. Procédé selon la revendication 2 dans lequel les monosaccharides sont choisis parmi le glucose, le mannose et le fructose, pris seuls ou en mélange.

4. Procédé selon la revendication 2 dans lequel les oligosaccharides sont choisis parmi le saccharose, le lactose, le maltose, l'isomaltose, l'inulobiose, le mélibiose, le gentiobiose, le tréhalose, le cellobiose, le cellotriose, le cellotetraose et les oligosaccharides issus de l'hydrolyse de polysaccharides issus de l'hydrolyse de l'amidon, de l'inuline, de la cellulose ou de l'hémicellulose, pris seuls ou en mélange.

5. Procédé selon l'une quelconque des revendications 1 à 4, dans lequel R est un groupement alkyle linéaire comportant de 1 à 6 atomes de carbone.

6. Procédé selon la revendication 5, dans lequel R est le groupement méthyle et le catalyseur de la famille des acides sulfoniques homogènes est l'acide méthanesulfonique.

7. Procédé selon l'une quelconque des revendications 1 à 4, dans lequel R est un groupement alkyle ramifié comportant de 3 à 10 atomes de carbone.

8. Procédé selon l'une quelconque des revendications 1 à 4, dans lequel R est un groupement aryle substitué et le catalyseur de la famille des acides sulfoniques est l'acide paratoluènesulfonique.

9. Procédé selon l'une quelconque des revendications précédentes, dans lequel le solvant polaire aprotique est choisi parmi l'acétone, le *N,N-*diméthylformamide, le *N,N*-diméthylacétamide, le sulfolane, la *N*-méthylpyrrolidone, le diméthylsulfoxyde, le propylène carbonate et la γ-valérolactone seuls ou en mélange.

10. Procédé selon l'une quelconque des revendications précédentes, dans lequel la température est comprise entre 50°C et 200°C, et dans lequel la pression est comprise entre 0,1 MPa et 8 MPa.

11. Procédé selon l'une quelconque des revendications précédentes, dans lequel la charge est introduite à un rapport massique solvant/charge compris entre 0,1 et 200.

12. Procédé selon l'une quelconque des revendications précédentes, dans lequel les catalyseurs de la famille des acides sulfoniques homogènes sont introduits à un rapport massique charge/catalyseur(s) organique(s) compris entre 1 et 1000.

## Patentansprüche

1. Verfahren zur Umwandlung eines mindestens einen Zucker umfassenden Einsatzstoffs in 5-Hydroxymethylfurfural, bei dem man den Einsatzstoff in Gegenwart von mindestens einem aprotischen polaren Lösungsmittel mit einem in Debye (D) ausgedrückten Dipolmoment von mehr als 2,00, das aus Pyridin, Butan-2-on, Aceton, Essigsäureanhydrid, N,N,N,N-Tetramethylharnstoff, Benzonitril, Acetonitril, Methylethylketon, Propionitril, Hexamethylphosphoramid, Nitrobenzol, Nitromethan, N,N-Dimethylformamid, N,N-Dimethylacetamid, Sulfolan, N-Methylpyrrolidon, Dimethylsulfoxid und Propylencarbonat alleine oder als Gemisch ausgewählt wird, bei einer Temperatur zwischen 30 °C und 200 °C und einem Druck zwischen 0,1 MPa und 10 MPa mit einem oder mehreren Katalysatoren aus der Familie der homogenen Sulfonsäuren in Kontakt bringt, wobei die Katalysatoren aus den Verbindungen der allgemeinen Formel 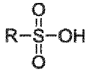 ausgewählt werden, wobei R aus
• Halogengruppen,
• linearen oder verzweigten, cyclischen oder acyclischen Alkylgruppen mit 1 bis 20 Kohlenstoffatomen, die gegebenenfalls durch mindestens einen Substituenten, der aus
∘ der Oxogruppe,
∘ Halogengruppen und
∘ gegebenenfalls kondensierten Arylgruppen, die gegebenenfalls durch Halogengruppen und/oder lineare oder verzweigte, cyclische oder acyclische Alkylgruppen mit 1 bis 20 Kohlenstoffatomen substituiert sein können,
ausgewählt ist, substituiert sein können,
• Arylgruppen mit 6 bis 14 Kohlenstoffatomen, die gegebenenfalls durch mindestens einen Substituenten, der aus
o linearen oder verzweigten, cyclischen oder acyclischen Alkylgruppen mit 1 bis 20 Kohlenstoffatomen, die gegebenenfalls durch mindestens eine Halogengruppe oder durch mindestens eine Nitrogruppe substituiert sein können,
∘ Halogengruppen und
∘ der Nitrogruppe
ausgewählt ist, substituiert sein können,
ausgewählt ist.

2. Verfahren nach Anspruch 1, wobei der Zucker aus Oligosacchariden und Monosacchariden alleine oder als Gemisch ausgewählt wird.

3. Verfahren nach Anspruch 2, wobei die Monosaccharide aus Glucose, Mannose und Fructose alleine oder als Gemisch ausgewählt werden.

4. Verfahren nach Anspruch 2, wobei die Oligosaccharide aus Saccharose, Lactose, Maltose, IsoMaltose, Inulobiose, Melibiose, Gentiobiose, Trehalose, Cellobiose, Cellotriose, Cellotetraose und Oligosacchariden aus der Hydrolyse von Polysacchariden aus der Hydrolyse von Stärke, Inulin, Cellulose oder Hemicellulose alleine oder als Gemisch ausgewählt werden.

5. Verfahren nach einem der Ansprüche 1 bis 4, wobei R für eine lineare Alkylgruppen mit 1 bis 6 Kohlenstoffatomen steht.

6. Verfahren nach Anspruch 5, wobei R für die Methylgruppe steht und es sich bei dem Katalysator aus der Familie der homogenen Sulfonsäuren um Methansulfonsäure handelt.

7. Verfahren nach einem der Ansprüche 1 bis 4, wobei R für eine verzweigte Alkylgruppe mit 3 bis 10 Kohlenstoffatomen steht.

8. Verfahren nach einem der Ansprüche 1 bis 4, wobei R für eine substituierte Arylgruppe steht und es sich bei dem Katalysator aus der Familie der Sulfonsäuren um para-Toluolsulfonsäure handelt.

9. Verfahren nach einem der vorhergehenden Ansprüche, wobei das aprotische polare Lösungsmittel aus Aceton, *N,N*-Dimethylformamid, *N,N*-Dimethylacetamid, Sulfolan, N-Methylpyrrolidon, Dimethylsulfoxid, Propylencarbonat und γ-Valerolacton alleine oder als Gemisch ausgewählt wird.

10. Verfahren nach einem der vorhergehenden Ansprüche, wobei die Temperatur zwischen 50 °C und 200 °C liegt und wobei der Druck zwischen 0,1 MPa und 8 MPa liegt.

11. Verfahren nach einem der vorhergehenden Ansprüche, wobei der Einsatzstoff mit einem Massenverhältnis von Lösungsmittel zu Einsatzstoff zwischen 0,1 und 200 eingetragen wird.

12. Verfahren nach einem der vorhergehenden Ansprüche, wobei die Katalysatoren aus der Familie der homogenen Sulfonsäuren in einem Massenverhältnis von Einsatzstoff zu organischem Katalysator bzw. organischen Katalysatoren zwischen 1 und 1000 eingetragen werden.

## Claims

1. Process for transforming a feedstock comprising at least one sugar into 5-hydroxymethylfurfural, in which said feedstock is placed in contact with one or more catalysts of the homogeneous sulfonic acid family in the presence of at least one polar aprotic solvent whose dipole moment expressed in Debyes (D) is greater than 2.00, chosen from pyridine, 2-butanone, acetone, acetic anhydride, N,N,N',N'-tetramethylurea, benzonitrile, acetonitrile, methyl ethyl ketone, propionitrile, hexamethylphosphoramide, nitrobenzene, nitromethane, N,N-dimethylformamide, N,N-dimethylacetamide, sulfolane, N-methylpyrrolidone, dimethyl sulfoxide and propylene carbonate, taken alone or as a mixture, at a temperature of between 30°C and 200°C, and at a pressure of between 0.1 MPa and 10 MPa, in which said catalysts are chosen from the compounds of general formula 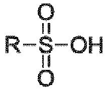 in which R is chosen from
• halogen groups,
• linear or branched, cyclic or non-cyclic alkyl groups comprising from 1 to 20 carbon atoms which may or may not be substituted with at least one substituent chosen from:
∘ an oxo group,
∘ halogen groups, and
∘ fused or non-fused aryl groups, which may or may not be substituted with halogen groups and/or linear or branched, cyclic or non-cyclic alkyl groups including from 1 to 20 carbon atoms,
• aryl groups comprising from 6 to 14 carbon atoms which may or may not be substituted with at least one substituent chosen from:
∘ linear or branched, cyclic or non-cyclic alkyl groups comprising from 1 to 20 carbon atoms which may or may not be substituted with at least one halogenated group or with at least one nitro group,
∘ halogenated groups, and
∘ a nitro group.

2. Process according to Claim 1, in which said sugar is chosen from oligosaccharides and monosaccharides, alone or as a mixture.

3. Process according to Claim 2, in which the monosaccharides are chosen from glucose, mannose and fructose, taken alone or as a mixture.

4. Process according to Claim 2, in which the oligosaccharides are chosen from sucrose, lactose, maltose, isomaltose, inulobiose, melibiose, gentiobiose, trehalose, cellobiose, cellotriose, cellotetraose and oligosaccharides derived from the hydrolysis of polysaccharides derived from the hydrolysis of starch, inulin, cellulose or hemicellulose, taken alone or as a mixture.

5. Process according to any one of Claims 1 to 4, in which R is a linear alkyl group including from 1 to 6 carbon atoms.

6. Process according to Claim 5, in which R is a methyl group and the catalyst of the homogeneous sulfonic acid family is methanesulfonic acid.

7. Process according to any one of Claims 1 to 4, in which R is a branched alkyl group including from 3 to 10 carbon atoms.

8. Process according to any one of Claims 1 to 4, in which R is a substituted aryl group and the catalyst of the sulfonic acid family is para-toluenesulfonic acid.

9. Process according to any one of the preceding claims, in which the polar aprotic solvent is chosen from acetone, N,N-dimethylformamide, N,N-dimethylacetamide, sulfolane, N-methylpyrrolidone, dimethyl sulfoxide, propylene carbonate and γ-valerolactone, alone or as a mixture.

10. Process according to any one of the preceding claims, in which the temperature is between 50°C and 200°C, and in which the pressure is between 0.1 MPa and 8 MPa.

11. Process according to any one of the preceding claims, in which the feedstock is introduced in a solvent/feedstock mass ratio of between 0.1 and 200.

12. Process according to any one of the preceding claims, in which the catalysts of the homogeneous sulfonic acid family are introduced in a feedstock/organic catalyst(s) mass ratio of between 1 and 1000.
